# EUROPEAN PATENT APPLICATION

(11) **EP 4 382 121 A1**
(43) Date of publication of application: **12.06.2024**
(21) Application number: 22306817.2
(22) Date of filing: 07.12.2022
(51) Int. Cl.: A61K 39/00, C07K 7/06, A61K 35/00

(54) **VACCINES AGAINST PTPRZ1-POSITIVE TUMORS**

(71) Applicant: Altevax, 75116 Paris (FR); Assistance Publique Hôpitaux de Paris, 75012 Paris (FR); Université Paris Cité, 75006 Paris (FR)
(72) Inventor: CARPENTIER, Antoine, 75007 Paris (FR); BANISSI, Claire, 94300 Vincennes (FR)
(74) Representative: Flesselles, Bruno F.G.

(57) **Abstract**

The invention relates to a novel epitope of PTPRZ1 which presents good immunogenicity and is useful for immunotherapy of cancer.

## Description

The invention pertains to the field of cancer treatment, and to the identification, modification and formulation of a specific peptide useful for immunotherapy of cancer.

PTPRZ1 is a receptor of the protein tyrosine phosphatase (PTP) family expressed within the cerebral tissues including cerebral cortex, cerebellum, hippocampus and caudate nucleus. PTPRZ1 is located in the glials and neuronal cells.

The structure of PTPRZ1 is made of a N-terminal carbonic anhydrase domain, a fibronectin type III domain, a domain for chondroitin sulfate (CS) attachment, a transmembrane segment, two phosphatase domains, and a C-terminal PDZ binding motif. The extracellular region of PTPRZ1 is responsible for its binding to various ligands (such as PTN, MK, IL-34 and basic fibroblast growth factor), extracellular matrix proteins (like tenascin C and R), and cell adhesion molecules. CS chains consist of repeated disaccharide units of GlcA and GalNAc with sulfate groups at various positions on sugar chains. Due to electrostatic repulsion between negatively charged glycosaminoglycan moieties, PTPRZ1 stay as monomers. PTN and MK have positive charged regions that, after binding, reduce the electrostatic repulsion of CS chains, thereby causing the dimerization and the inactivation of the phospatase activity. PTPRZ1, through binding to its ligands, also plays a role in differentiation, cell motility and myelination, and might play a role in demyelinating processes such as multiple sclerosis. The interaction of PTPRZ1 and PTN also have an impact on tumor microenvironment, namely M2 tumor associated macrophages.

In cancer, and especially in gliomas, PTPRZ1 is sometimes involved in fusion genes (translocation); Twenty five PTPRZ1 fusion genes have thus been described in various cancers (Woo et al, 2020). Gene fusions (PTPRZ1-MET, PTPRZ1-ETV1...), are frequently reported in gliomas (Woo et al, 2020).

Independently of fusion genes in cancer, several splice variants of PTPRZ1 have been described; the long transmembrane form of 9.5 kb; the short transmembrane form of 6.4 kb (deleted in the extracellular region), and 2 soluble secreted forms, one short (4 kb) and one long (8.4 kb), also known as phosphacans. The transcripts are differentially expressed depending on cell types (Cannol 1996).

PTPRZ1 expression is particularly high in gliomas, present in melanomas, lung and neck carcinomas, and lower in other types of cancers. Immunohistochemistry shows that PTPRZ1 is widely expressed throughout the glioma tissue both at the nuclear and the cytoplasmic level. Expression of PTPRZ1 was reported in 16 of the 24 glioblastomas (66%), with co-expression of long-PTPRZ1 and short-PTPRZ1 in almost all positive glioma samples (14/16) (Lorente et al, 2005). In glioma, PTPRZ1 is overexpressed in glioma stem cells (GSCs) compared to non- tumor cells (Shy et al, 2017). Antibodies targeting PTPRZ1 have shown remarkable antitumor properties in several glioma models (Foehr et al, 2006; Shy et al, 2017), and knock-down experiments further support the role of PTPRZ1 in glioma oncogenesis (Fujikawa et al, 2017).

PTPRZ1 thus appears as a relevant antigenic target for cancer vaccine. Hilf et al (2019) disclosed epitope PTP-013 (MIWEHNVEV, SEQ ID NO: 4) and the fact that this epitope is naturally presented by HLA-A2 of patients. In the APVAC trial conducted in glioblastomas, reactive CD8+ T cells against this peptide were found in some patients after immunization of patients. This peptide, derived from PTPRZ1 thus showed to be an epitope able to generate an immune response. In another trial (IMA950), patients mounted CD8 T-cell responses against various tumor peptides, including one derived from PTPRZ1. However, the immune responses were weak and did not translate in clinical efficacy (Migliorini et al, 2019). Other PTPRZ1 human epitopes have been described in the literature (Dutoit 2012; Neidert 2018).

An optimal PTPRZ1 epitope to immunize patients against PTPRZ1-positive tumors shall preferably present the two following properties:
- the epitope must be strongly immunogenic in humans, i.e. can induce a strong immune response after immunization; and
- the epitope should be located on regions shared by the long and the short isoforms, otherwise the tumor might escape the immune response by shutting down the expression of the long isoform.

Priming lymphocytes or antibodies against a specific target (a process named immunization) result from the presentation of one antigen by antigen-presenting cells to T cells. This process can be achieved *in vitro,* but is more easily achieved *in vivo,* by administration of the antigen into living animals or humans (a procedure named vaccination). Vaccines, despites all the progress made, are still facing several limitations. Most antigens are poorly immunogenic. The dose of a peptidic antigen required to trigger the immunity (usually within the range of 10 to 300µg) might be a limiting factor, especially when antigen is difficult to manufacture, or when demand exceeds production capacity. Moreover, induction of CD8+ lymphocytes remains a difficult challenge because antigen injected extracellularly are usually presented by MHC class II and not by MHC class I (thus preferentially leading to CD4+ lymphocytes and antibodies). Finally, vaccine technologies, such as emulsion, liposomes, nanoparticles, fusion molecules, or DNA and RNA vaccines can be either unstable or difficult to synthesize, making the cost of manufacturing sometimes prohibitive. Adjuvants, are generally used for increasing the immunogenicity of the antigen administered.

WO2017089529 discloses that melanin can be used as an adjuvant to increase immune response against an antigen bearing epitopes.

WO2021165306 discloses that addition of an amino acid with a nucleophilic residue to a peptide is useful to improve immunogenicity of the peptide when complexed with melanin. Preferred amino acids are cysteine, acetylcysteine, methionine, proline, hydroxyproline, histidine and lysine, with cysteine being of particular interest.

The inventors modified the epitope PTP-013 disclosed in Hilf, by mutating one of its residues and showed that this mutation increases the immune response generated when administered *in vivo,* and that the T-lymphocytes from animals immunized with this modified epitope can be re-stimulated with the non-modified peptide, thereby demonstrating cross-reactivity. The inventors showed that the modified epitope generates a strong immune response when complexed with melanin, by itself, and especially after being modified by addition of two lysines at its N-terminus. The inventors showed that, using melanin as an adjuvant, the immunogenicity was improved when the epitope was modified, as compared to the unmodified epitope (even when it is modified according to WO2021165306 by addition of cysteine, lysine or histidine at its end-terminus). Notably, addition of two lysines at the end-terminus of the modified epitope led to a very strong immune response.

In a first embodiment, the invention thus relates to a polypeptide or a peptide bearing this specific epitope. The invention this relates to a polypeptide or a peptide comprising SEQ ID NO: 15, or a peptide consisting in SEQ ID NO: 15.

In another embodiment, the invention relates to a polypeptide or a peptide which comprises SEQ ID NO: 14, or which consists in SEQ ID NO: 14.

Both SEQ ID NO: 15 and SEQ ID NO: 14, which comprises SEQ ID NO: 15, can be used to generate an immune response against PTPRZ1 that is useful for the treatment of cancers.

As indicated below, the peptide comprises preferably at most 100, more preferably at most 50 amino acids. When the peptide is bigger than SEQ ID NO: 14 (resp. SEQ ID NO: 15), SEQ ID NO: 14 (resp. SEQ ID N: 15) may be located at the N-terminus of the peptide, or somewhere else within the peptide. The peptide may however contain more than 50 or 100 amino acids. It may be a protein.

In some embodiments, the polypeptide or peptide that comprises SEQ ID NO: 14 or SEQ ID NO: 15 further comprises CD4 or CD8 epitopes of antigens, in particular of cancer antigens, notably the cancers targeted by the immunogenic compositions herein disclosed. The other epitopes can be linked to SEQ ID NO: 14 or SEQ ID NO: 15 by stretches of amino acids (this is the preferred embodiment) or any other acceptable linkers such as polyether compounds or other linkers used in dendrimer constructs. When melanin is used as an adjuvant, it is preferred when SEQ ID NO: 14 or SEQ ID No: 15 is at the N-terminus of the polypeptide or peptide.

The other epitopes may be other epitopes from PTPRZ1 or epitopes from other proteins than PTPRZ1, the expression of which is associated with the same cancers than PTPRZ1.

The other epitopes may be universal T helper epitopes such as pan-DR epitope (PADRE) and PoI₇₁₁ epitopes. The literature widely discloses other universal T helper epitopes. This can improve the immune response against cells expressing PTPRZ1 *via* the response against SEQ ID NO: 15.

The invention also relates to a nucleic acid molecule coding for the polypeptides or peptides disclosed above. In some embodiments, the nucleic acid molecule is DNA. In other embodiments, the nucleic acid molecule is RNA. In some embodiments, the nucleic acid molecule is a chimera DNA-RNA. The nucleic acid may be protected from degradation, when administered to a subject, by methods known in the art, in particular by liposome encapsulation. It may be very advantageous to introduce a nucleic acid coding for SEQ ID NO: 15 within a larger DNA or RNA sequence, that is to be used as a vaccine, and that would also code for other epitopes or for a whole protein. Such constructs are included within the scope of the invention.

The polypeptide or peptide herein disclosed can be used for treating or preventing cancers where PTPRZ1 expression is high, and notably glioblstomas, gliomas, melanomas, lung and head-and-neck, cervical or testis cancers.

The invention thus relates to the polypeptide or peptide herein disclosed, the nucleic acid molecule or the vaccine composition disclosed below for use as a medicament, and in particular as a vaccine (whether prophylactic or therapeutic).

The invention thus also relates to a vaccine composition (or an immunogenic composition) comprising the polypeptide, peptide or the nucleic acid molecule herein disclosed. In the vaccine composition, the polypeptide, peptide or the nucleic acid molecule is formulated with appropriate excipients and optionally some adjuvants to be injected to a mammal, notably a human being. In particular, the administration can be an intramuscular, intravenous, intraperitoneal injection, or an injection directly into the tumor. In other embodiments, the vaccine composition can take the form of an oral, inhalable or intradermal composition, notably in the form of a patch.

It is preferred when the vaccine composition comprises a polypeptide or peptide herein disclosed, and an adjuvant, and in particular when the adjuvant is melanin. One shall preferably use a synthetic melanin (i.e. a melanin obtained *in vitro* from oxidative polymerization of a precursor) melanin. In particular, the melanin is a soluble melanin. In this embodiment, the peptide shall be complexed to the melanin. The vaccine composition can be obtained by oxidative polymerization of a melanin precursor in the presence of the peptide, as disclosed in WO2017089529, or by addition of the peptide to an already synthetized melanin as disclosed in WO2021165306.

The invention also relates to the polypeptide, peptide, the nucleic acid or the vaccine composition herein disclosed for use for the treatment of cancer.

The invention also relates to the use of a polypeptide, peptide, a nucleic acid or an immunogenic composition herein disclosed for the preparation of a medicament for the prevention or treatment of a cancer in a patient. In this embodiment, the medicament contains the polypeptide, peptide, the nucleic acid or the immunogenic composition as well as appropriate excipients or adjuvants.

The vaccine may be a prophylactic (i.e. intended to protect the recipient against the development of a disease) or a therapeutic (i.e. intended to help the recipient fight an already present disease) vaccine. The disease is linked to the target antigen (PTPRZ1), expressed or presented by cells during the course of the disease.

The invention also relates to a method for treatment of a patient in need thereof (in particular a patient with a cancer), comprising administering an effective amount of the polypeptide, peptide, nucleic acid or immunogenic composition herein disclosed to the patient. Such administration leads to the generation of an immune response directed against PTPRZ1, which would then attack and eliminate the tumor cells, thereby providing a therapeutic effect.

The invention also relates to a method for protecting a patient against a cancer, comprising administering a therapeutic or effective amount of a polypeptide, a peptide, a nucleic acid or an immunostimulatory composition as disclosed herein to the patient, so as to induce an immune response against PTPRZ1 that is associated with the cancer, wherein the immune response has a protective (prophylactic) effect against the cancer.

In particular, the cancer is a low or high grade glial tumor.

In another embodiment, the cancer is a melanoma.

In another embodiment, the cancer is a head-and-neck cancer.

In another embodiment, the cancer is a lung cancer.

In another embodiment, the cancer is a cervical cancer.

In another embodiment, the cancer is a testis cancer.

An "effective amount" or a "therapeutic" of an agent, as used herein, is the amount sufficient to induce beneficial or desired results, such as clinical results or onset of an immune response, in particular a T-cell mediated immune response. In the present context, a therapeutic amount of an agent is, for example, an amount sufficient to achieve onset of an immune response against the antigen, and reduction in the severity of a symptom of the disease linked to the antigen, as compared to the situation observed without administration of the composition. An effective amount is an amount that provides therapeutic improvement while minimizing side or adverse effect. One can use, as effective amounts, 10 µg to 5 mg of antigen, preferably between 100 µg and 500 µg. In case melanin is used in the vaccine formulation, the amount of melanin that can be used may be comprised between 50 µg and 10 mg, in particular between 500 µg and 2 mg or between 200 µg and 1 mg.

The resulting vaccine formulation can be used to protect an animal against a disease implicating (i.e. involving and/or concerning) cells that are expressing inside, at their surface, or secreting PTPRZ1.

Alternatively, the polypeptide, peptide, nucleic acid or immunostimulatory composition can be used *in vitro* in presence of live cells (for example macrophages, dendritic cells, antigen-presenting cells or lymphocytes), to sensitize them to the antigen, for instance before administration (preferably injection) in humans (priming). The resulting composition will thus elicit an immune response against the antigen PTPRZ1 in the recipient. In particular, US 6210662 discloses such principle of forming therapeutic or immunogenic compositions consisting of antigen presenting cells activated by contact with an antigen complex.

The invention thus relates to an *in vitro* method for priming or stimulating CD8 lymphocytes against PTPRZ1, comprising contacting and incubating a polypeptide, a peptide or a nucleic acid molecule or an immunogenic composition herein disclosed, optionally with an adjuvant, with antigen presenting cells and T-lymphocytes. It is preferred when the antigen presenting cells and T-lymphocytes have been isolated from a patient with cancer. The resulting lymphocytes can then be recovered and administered to a patient for the treatment of cancer involving PTPRZ1.

The invention also relates to a method to detect an immune response against PTPRZ1 (in particular against the epitope depicted by SEQ NO: 15) by co-incubation of an immunostimulatory composition as disclosed herein with a patient's tissue containing immune cells (such as blood, or isolated lymphocytes) and detecting a specific immune response of the patient's lymphocytes against the PTPRZ1 antigen present in the immunostimulatory composition. Such response can be detected by measure of molecules secreted by the lymphocytes, such as cytokines and in particular gamma-interferon.

The invention also relates to a method to detect an immune response against PTPRZ1 (in particular against the epitope depicted by SEQ NO: 15) by co-incubation of a population of antigen presenting cells (such as monocytes, or dendritic cells) with a patient's tissue containing immune cells (such as blood, or isolated lymphocytes) and detecting a specific immune response of the patient's lymphocytes against the PTPRZ1 antigen present in the immunostimulatory composition. Such response can be detected by measure of molecules secreted by the lymphocytes, such as cytokines and in particular gamma-interferon. The antigen presenting cells have previously been incubated in the presence of the immunogenic composition herein disclosed, and thus present the epitome depicted by SEQ ID NO: 15 at their surface by the MHC I molecules.

These methods are performed *in vitro.*

### Melanin

It is reminded that a "melanin" is a pigment being a macromolecule obtained from the oxidative polymerization of precursors related to indole or cathecol (generally starting with the oxidation of the amino acid tyrosine (or another precursor), followed by polymerization. This oxidation is a critical step and is generally mediated by the enzyme tyrosinase, which will convert tyrosine to DOPA. WO2017089529 and WO2021165306 disclose the process of synthesis of eumelanin. As a melanin usable as an adjuvant with the epitope herein disclosed, on can use a "natural" melanin, as could be found in nature, such as an eumelanin, a MAPs-like polymer (containing a high proportion of a melanin precursor), or a synthetic melanin molecule that is obtained by *in vitro* oxidative polymerization of precursor derivatives such as the ones described below. Synthetic melanin, prepared in particular by oxidation of tyrosine with hydrogen peroxide, is thus sold as such, for instance by Sigma Aldrich.

A preferred melanin, in the context of the present application, is eumelanin.

### Obtaining a synthetic melanin

A synthetic melanin is obtained after oxidative polymerization of melanin precursors *in vitro.*

Polymerization of melanin precursors can be performed by methods known in the art. In particular, the melanin precursor may be incubated, with or without buffer, with an enzyme such as phenylalanine hydroxylase, tyrosinase, mushroom tyrosinase, tyrosine hydroxylase, peroxidase, phenol-oxidase, Dopachrome tautomerase, DHICA oxidase, DHI oxidase. The choice of the enzyme will be made by the person skilled in the art depending on the nature of the precursor present in solution before polymerization. It is favored when the oxidative polymerization is performed in presence of tyrosinase.

The mixture is also exposed to an oxidizing agent (oxidant or oxidative agent) as disclosed below in order to promote the polymerization and obtain the synthetic melanin.

Among others, the person skilled in the art may optimize various parameters such as the ratio of melanin precursors is a mixture is used, the type of oxidant, pH, buffer, length of incubation, or temperature of reaction.

In particular, melanin synthesis may be influenced by pH (alkaline pH promoting auto-oxidation of catechol), and presence of metal ions (such as Cu²⁺, Ni²⁺, Fe³⁺, Fe²⁺, Co²⁺, Zn²⁺, Mn²⁺, Mg²⁺...) present in the incubation solution (Palumbo et al, Biochim Biophys Acta. 1987; 13;925(2):203-9; Palumbo et al, Biochim Biophys Acta. 1991;1115(1):1-5; WO95009629). Thus, working at a pH 8.5+/-0.5 is adapted. Physico-chemical conditions can be modified to increase the reaction kinetics, such as increasing temperature above 20°C (for example between 60 and 80°C), of bubbling air into the reaction mixture, or increasing the atmospheric pressure.

Such synthetic melanin may be more homogeneous than natural melanin and could thus be distinguished from it. It is preferred, in the context of the present invention, to use a synthetic eumelanin-like, in particular obtained from *in vitro* oxidative polymerization of L-Dopa.

In an embodiment, the synthetic melanin (post polymerization) is purified by filtration on a 5kDa-100kDa filter, preferably a 10kDa filter.

In a preferred embodiment, the synthetic melanin is a soluble melanin, i.e. is in the form of particles of less than 500nm.

When synthetized, melanin is washed by ultrafiltration or by filtration on an approximately 10kDa filter (melanin remains on the retentate), then resuspended in in water or buffer (such as a phosphate buffer). Melanin can be filtered through a 0.2 µm filter for sterility. Thus, in an embodiment, the melanin is resuspended in water with or without buffer (such as phosphate buffer) prior to being mixed with the peptide to obtain an immunogenic composition

### Melanin Precursor

A "melanin precursor" is a molecule that is used or synthetized during the synthesis of a melanin, in particular a eumelanin, *in vitro.* One can cite: L-phenylalanine, L-tyrosine, L-dopa, dopaquinone, cyclodopa, dopachrome, Dihydroxyindole carboxylic acid or 5,6-dihydroxyindole-2carboxylic acid (DHICA), indol 5,6 quinone, 5,6-dihydroxyindole (DHI), dopamine-o-quinone, Dopamine leukodopaminochrome, leukodopachrome (cyclodopa), dopaminochrome, norepinephrine, noradequinone, noradenochrome, epinephrine, epinephrine-o-quinone, adenochrome, 3-amino-tyrosine, 6-hydroxy-Dopa, dihydrocaffeic acid, caffeic acid, methide, benzothiazole, benzothiazine, dihydroesculetin and others.

Indeed, the term "melanin precursor" further includes derivatives of such precursors and/or polymers containing a high proportion of such precursors (such as in Mussel Adhesives Proteins). Such melanin precursors and derivatives are described in WO2017089529 (incorporated by reference with regards to this teaching) and can be used as equivalent melanin precursors in the context of the present invention.

The melanin precursor is preferably selected from the group consisting of DHICA, DHI, L-dopa, L-tyrosine, D-dopa, 6-hydroxy-Dopa, dopaquinone, cyclodopa, dopachrome, dopamine-o-quinone, dopamine, leukodopaminochrome and dopaminochrome.

A preferred melanin precursor is L-dopa. Another preferred melanin precursor is DHICA. Another preferred melanin precursor is DHI. Another preferred melanin precursor is L-tyrosine. In a specific embodiment, the melanin precursor is a mixture of DHICA and DHI. In another embodiment, the melanin precursor is dopachrome.

Other melanin precursors or derivatives thereof are described in the art, such as the products described in WO2017089529.

### Oxidizing agent

An "oxidizing agent" or "oxidizing molecule" is a compound that is able to provide oxygen to a solution containing melanin precursors and promote polymerization thereof and formation of a melanin macromolecule.

Oxidizing agents that can achieve this goal comprise oxygen, hydrogen peroxide, ammonium persulfate, ferric ions, sodium iodide together with hydrogen peroxide, and treatment with a salt of a transition metal cation such as copper sulfate as a catalyst for air oxidation.

It is thus preferred when the oxidizing agent is chosen in the group consisting of oxygen, hydrogen peroxide, ammonium persulfate, and ferric ions.

### Vaccine, immunogenic or immunostimulatory composition

An "vaccine, immunogenic or immunostimulatory composition" is a composition that is able to generate an immune response in an animal when administered to said animal. Preferably, said animal is a mammal, but is can also be a bird (such as a chicken, a duck, a goose, a turkey, a quail), in particular when the composition is used in avian livestock. The animal may also be a fish, as the immunogenic composition may be used in fish farming.

However, the immunogenic composition is preferably used in mammals. Such mammals are preferably human beings, but can also be other mammals, when the composition is used in the veterinary field, in particular for inducing immunity in livestocks such as cattle (cows), sheeps, goats or horses, but also for pets such as dogs or cats.

The immunogenic composition is thus a composition that contains an antigen, in particular a peptide containing epitopes from an antigen, and that is able to generate an immune response against such antigen. The generated immune response can be a cellular (T-cell mediated) or a humoral (B-cell mediated, production of antibodies) immune response. The immunogenic composition may also induce both a cellular and a humoral immune response.

The cellular immune response can be a CD8 T lymphocytes mediated response (ie cytotoxic response), or a CD4 T lymphocytes mediated response (helper response). It can also combine a cytotoxic and helper cellular immune response. The helpher response may involve Th1, Th2 or Th17lymphocytes (such lymphocytes being able to elicit different cytokine responses, as is known in the art).

The immunogenic composition may allow a better presentation of the antigen present therein, through MHC1 or MHC2 pathways.

The immunogenic composition shall contain a polypeptide or a peptide comprising SEQ ID NO: 14 or SEQ ID NO: 15. It may also be a mixture of polypeptides or peptides.

In some embodiments, the immunogenic composition contains a nucleic acid coding for a polypeptide or a peptide comprising SEQ ID NO: 14 or SEQ ID NO: 15. Administration of this immunogenic composition makes it possible to obtain expression of the polypeptides or peptides within cells *in vivo,* ad obtaining an immune response against these polypeptides or peptides. Cells may also be transfected *in vitro* by the nucleic acid coding for a polypeptide or a peptide comprising SEQ ID NO: 14 or SEQ ID NO: 15, and the resulting cell composition can be used as the immunogenic composition.

### Adjuvant

An "adjuvant" is a substance that has the capacity to modify or enhance the immune response to an antigen. In other words, the immune response against the antigen may be higher or different in the presence of the adjuvant than when the adjuvant is not present (that includes when the response is modified, for example when the subset of T cells that are activated in the presence of the adjuvant is different from the subset activated in the absence of the adjuvant). A lot of adjuvants are known in the art and have been widely used in the vaccine field.

One can cite alum, emulsions (either oil-in-water or water-in-oil, such as Freund's Incomplete Adjuvant (IFA) and MF59^{®}), PRR (Pattern recognition receptors) Ligands, TLR3 (Toll-Like Receptor 3) and RLR (RIG-I Like Receptors) ligands such as double-stranded RNA (dsRNA), or synthetic analogs of dsRNA, such as poly(I:C), TLR4 ligands such as bacterial lipopolysaccharides (LPS), MPLA (monophosphoryl lipid A), in particular formulated with alum, TLR5 ligands such as bacterial flagellin, TLR7/8 ligands such as imidazoquinolines (i.e. imiquimod, gardiquimod and R848), TLR9 ligands such as oligodeoxynucleotides containing specific CpG motifs (CpG ODNs) or NOD2 (Nucleotide-binding oligomerization domain-containing protein 2) ligands. The term ligand above describes preferably an agonist of the receptor, *i.e.* a substance that binds to the receptor and activates the receptor, in particular for TLR3 and TLR9 receptors. Melanin as herein described, acts as an adjuvant and another adjuvant may also be used.

When melanin is used and another adjuvant is added, it is preferred when it is selected in the group consisting of TLR3 agonists and TLR9 agonists and in particular when this adjuvant that is further added is chosen among Polyinosinic:polycytidylic acid (poly I:C) and CpG oligonucleotides.

### Peptide

A peptide is a chain of amino acids linked by peptide bonds. In the context of the invention, a peptide shall comprise at least 9 amino acids, more preferably at least 10 amino acids, more preferably at least 11 amino acids, or at least 12 amino acids. In some embodiments, the peptide shall contain at most 100 amino acids, more preferably at most 50 amino acids, more preferably at most 30 amino acids, more preferably at most 25 amino acids. Peptides between 10 and 25 amino acids are well adapted. In other embodiments though, the peptide (which may be referred to as a polypeptide) may contain more than 100 amino acids. It may be a protein.

One or more amino acids of the peptide may be artificial (different from one of the 20 amino acids found in proteins in nature). Such artificial amino acid may be a D-amino acid, or a non-natural amino acid (such as citrulline, hydroxyproline, norleucine 3-nitrotyrosine, nitroarginine, ornithine, naphtylalanine...).

The peptide may be capped or modified at its N and/or C-terminus. In particular, acetylation or capping of the N-terminus helps to minimize amino peptidase degradation of the peptide when amidation of the C terminus helps to stabilize the peptide from carboxypeptidase degradation.

In the context of the invention, a biologically active peptide should thus contain the epitope depicted by SEQ ID NO: 15 or SEQ ID NO: 14. As indicated, these sequences might be included in a large protein or in a longer peptide, and further modified with glycosylation or extremities protection.

### Vaccine

In the context of the invention, a vaccine is a composition that is administered to an animal produce or artificially increase immunity to a particular antigen. It is thus undersood that the terms "immunogenic composition", "immunostimulatory composition" and "vaccine" can indifferently be used.

### Obtaining an immunogenic composition

An immunogenic composition can be obtained by combining a polypeptide or a peptide containing the modified epitope herein disclosed, with an adjuvant.

In particular, the polypeptide or peptide bearing the modified epitope is combined to a melanin, in particular a synthetic melanin as herein described.

Polypeptides or peptides can be added to a synthetic melanin solution as disclosed above (weight ratio polypeptide or peptide/melanin between 1/1 and 1/10) and incubated for various periods of time before usage, preferably at room temperature. The resulting solution can be washed and resuspended in water or in any appropriate buffer.

The binding of the polypeptide or peptide to the melanin can be verified by Tricine-SDS-PAGE analysis as described in Carpentier (2017). Briefly, samples (peptide-Mel or peptide alone) are loaded on acrylamide gels. Following electrophoresis, the gels are stained with Coomassie Brilliant Blue R-250, allowing the quantification of the free peptide in the gel. The binding of peptides to melanin can be expressed as the ratio: [amount of unbound peptide in samples Peptide-Mel / amount of peptides in control samples containing peptides alone.

The immunostimulatory composition may also comprise another adjuvant as disclosed above. In a preferred embodiment, the adjuvant is added to the composition obtained just before administration, i.e. less than one hour before administration.

### FIGURES

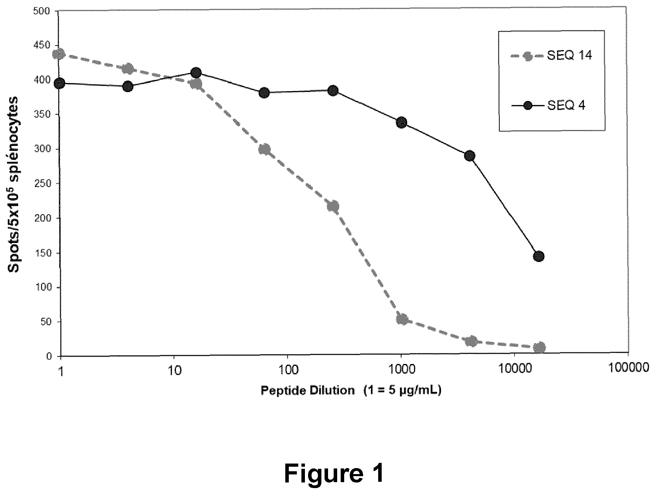

Figure 1: cross reactivity of T-cell responses obtained after immunizations with SEQ 14 in transgenic SURE mice. Mice were immunized against SEQ 14, as described in table 1, and sacrificed on day 8. The splenocytes (5.10^5 cells /well) were re-stimulated in vitro for 18 hours with either SEQ 14 or the native epitope SEQ 4 (both non-conjugated to melanin), at various concentrations (5 µg/ml to 0.3 ng/ml) and the numbers of IFNg-SFCs (Spot forming cells) were measured after a 18-hour incubation (representative experiments from 3 experiments)

### EXAMPLES

### Example 1. Screening of peptides

For the purpose of selecting a relevant PTPRZ1 antigen to be used in human patients, several peptides containing various PTPRZ1 immune epitopes were screened. The immunogenicity of these various peptides were screened by vaccinations in HLA-A2/DR1 mice (also known as Surel1 model). HLA-A2/DR1 mice are transgenic mice mimicking the human's immune system. As a vaccine procedure for screening, a melanin-based vaccine was used, as described in WO2021165306.

A few potential CD8 epitopes within the PTPRZ1 human sequence were identified (SEQ ID NO: 1 to SEQ ID NO: 4, table 1), as described in the literature (Dutoit, 2012, Hilf 2018, Neidert, 2018). Three of these 4 epitopes were located on regions shared by long and short PTPRZ1 isoforms

Using a melanin-based vaccine in HLA-A2/DR1 mice, these epitopes were then screened using peptides (SEQ ID NO: 5 to SEQ ID NO: 8) containing the epitope and an additional cysteine at the NH2-terminal, as it was disclosed in WO2021165306 that this modification increases the efficacy of melanin-based vaccine.

SEQ ID NO: 5 induced a powerful immune response but this epitope is not present in the short PTPRZ1 isoform.

SEQ ID NO: 8 is shared by the long and the short isoforms, and was able to trigger an immune response (Table 1).

Because the immune response obtained after immunization with SEQ 8 was weak, a few amino-acids were modified within its sequence at the NH2-terminus.

The immunogenicity of the modified peptides (SEQ ID NO: 9 to SEQ ID NO: 14) after immunization in mice was screened.

Surprisingly and unexpectedly, it was observed that peptide SEQ ID NO: 14 was the only one that triggered a strong CD8 immune response after immunization in mice (Table 1), despite the fact that the modification involved part of the epitope sequence itself (SEQ ID NO: 4).

**Table 1. list of sequences used, and the corresponding T-cell responses obtained after subcutaneous immunizations in transgenic Surel1 mice.**

| | **Peptidic sequence** | **T-cell Immune response** : **mean +/- SEM (number of mice)** |
|---|---|---|
| SEQ ID NO: 1¹ | KVFAGIPTV | |
| SEQ ID NO: 2² | VLDSHIHAY | |
| SEQ ID NO: 3³ | AllDGVESV | |
| SEQ ID NO: 4⁴ | MIWEHNVEV | |
| SEQ ID NO: 5 | CKVFAGIPTV | 288 +/-37 (n=8) |
| SEQ ID NO: 6 | C VLDSHIHAYVNALLIPGPAGK | 7 +/-5 (n=4) |
| SEQ ID NO: 7 | C AIIDGVESV | 3 +/-1 (n=4) |
| SEQ ID NO: 8 | C MIWEHNVEV | 24 +/-8 (n=8) |
| SEQ ID NO: 9 | K MIWEHNVEV | 63 +/ 12 (n=7) |
| SEQ ID NO: 10 | P MIWEHNVEV | 40 +/-15 (n=7) |
| SEQ ID NO: 11 | H MIWEHNVEV | 60 +/-25 (n=7) |
| SEQ ID NO: 12 | PK MIWEHNVEV | 61 +/-30 (n=7) |
| SEQ ID NO: 13 | KP MIWEHNVEV | 80 +/-34 (n=7) |
| SEQ ID NO: 14 | KK KIWEHNVEV | 287 +/-26 (n=8) |
| SEQ ID NO: 15 | KIWEHNVEV | Not yet available |

| | | |
|---|---|---|
| 1. Described in Dutoit 2012 and Hilf 2018. Not included in the short isoform 2. Described in Neidert 2018. Located in the intracellular part of PTPRZ1 3. Described in Dutoit 2012, and Hilf 2018 4. Described in Hilf 2018. Located in the intracellular part of PTPRZ1 | | |

For immunizations, L-Dopa (0.8mg/ml) underwent an oxidative polymerization at pH 8.5 in aerobic conditions for 2 hours at 60°C. The reaction mixture was then filtered on a 10kDa filter, and the retentate containing the synthetic melanin was resuspended in phosphate buffer. Peptides (10 µg/mouse) containing the epitopes (underlined) were then added (at the weight ratio peptide/L-Dopa =1/4) and the mixtures were used for subcutaneous immunizations in mice. Phosphorothioate oligonucleotide CpG-28 (5'-TAAACGTTATAACGTTATGACGTCAT, SEQ ID NO: 16) was added to vaccine formulations (10 µg/mouse) just before the immunizations. Mice were sacrificed on day 8 and the CD8 T-cell response was performed as described in Carpentier; 2017. Briefly, splenocytes were re-stimulated in vitro with the corresponding MHC class I-epitope (non-conjugated to melanin) and the numbers of IFNg-SFCs (Spot forming cells) were measured and expressed as Mean+/-S.E.M.

### Example 2. Verification of the cross-reactivity of the novel epitope

As SEQ ID NO: 14 is modified from the natural epitope SEQ ID NO: 4, one can expect that this modification may result in a totally new epitope, having no cross reactivity with the native epitope.

Mice were thus immunized with SEQ ID NO: 14, and it was checked whether T-cell response triggered by SEQ ID NO: 14 was able to similarly recognize the mutated epitote SEQ ID NO: 15 and the native epitope SEQ ID NO: 4.

It was shown that this is the case (Figure 1), thus demonstrating cross reactivity of the immune response on both epitopes.

Surprisingly, the reactivity against the native epitope (SEQ ID NO: 4) of the lymphocytes primed with the modified epitope (SEQ ID NO: 14)was even higher that that against the modified epitope used to immunize the animals (SEQ ID NO: 14 or SEQ ID NO: 15).

The modification of an amino-acid within the epitope to make it more immunogenic, especially by increasing their affinity to MHC by changing amino acid located at the "anchor" places (that bind the epitope to the MHC class I, usually on the 2^{nd} and the 9^{th} position on a 9-mer epitope) have been of interest for many years (Hebeisen M et al. 2013), but no clear rules have been defined to optimize a given epitope.

However, in this application, the reactivity of primed lymphocytes against the native epitope (SEQ ID NO: 4) was higher than towards the mutated one (SEQ ID NO: 14), suggesting that the affinity of the later is lower (fig 1).

In line with these results, the modification described in SEQ ID NO: 14 or SEQ ID NO: 15 was outside the classical anchor places, and this modification is not supposed to increase the epitope affinity when tested on binding predictor software, such as the ones disclosed at the IEDB (Immune Epitope Database and Analysis resource), a resource funded by NIAID cataloging experimental data on antibody and T cell epitopes and hosting tools to assist in the prediction and analysis of epitopes. Available at http://tools.immuneepitope.org/mhci/ or the collaboration service between CBS, ISIM, and LIAI that makes epitopes can be made for peptides of any length. services.healthtech.dtu.dk/service.php?NetMHC-4.0.

This modification to SEQ ID NO: 4 thus gave totally unexpected results.

### REFERENCES

Canoll et al. (1996). Journal of neuroscience research, 44(3), 199-215. doi.org/10.1002/(SICI)1097-4547(19960501)44:3
Carpentier et al. (2017) PLoS One. Jul 17;12(7):e0181403. doi: 10.1371/journal.pone.0181403
Dutoit et al. (2012). Brain. Apr;135(Pt 4):1042-54. doi: 10.1093/brain/aws042. Foehr et al. (2006). Cancer research, 66(4), 2271-2278. doi.org/10.1158/0008-5472.CAN-05- 1221
Fujikawa et al. (2017). Scientific reports, 7(1), 5609. doi.org/10.1038/s41598-017-05931-8
Hebeisen M et al. 2013. Molecular Insights for Optimizing T Cell Receptor Specificity Against Cancer. Frontiers in Immunology 4Hilf et al. (2019). Nature, 565(7738), 240-245. doi.org/10.1038/s41586-018-0810-y
Lorente et al. (2005). Neuro oncology, 7 2 154-163. doi.org/10.1215/S1152851704000547
Migliorini et al. (2019). Neuro-oncology, 21(7), 923-933. doi. org/10.1093/neuonc/noz040
Neidert et al. (2018). Acta Neuropathol. Jun;135(6):923-938. doi: 10.1007/s00401-018-1836-9. Epub 2018 Mar 20.
Palumbo et al, Biochim Biophys Acta. 1987; 13;925(2):203-9;
Palumbo et al, Biochim Biophys Acta. 1991;1115(1):1-5;
Shi et al. (2017). Nature communications, 8, 15080. doi.org/10.1038/ncomms15080
Woo et al. (2020). Brain tumor pathology, 37(4), 136-144. doi.org/10.1007/s10014-020-00377-9
WO95009629
WO2017089529
WO2021165306

## Claims

1. A peptide comprising SEQ ID NO: 15.

2. The peptide of claim 1, which consists in SEQ ID NO: 15.

3. The peptide of claim 1, which comprises SEQ ID NO: 14.

4. The peptide of claim 3, which consists in SEQ ID NO: 14.

5. The peptide of claim 1 or 3, which comprises at most 50 amino acids.

6. The peptide of any one of claims 1 or 3 or 5, which further comprises epitopes of cancer antigens.

7. A nucleic acid molecule coding for the peptide of any one of claims 1 to 6.

8. A vaccine composition comprising the peptide of any one of claim 1 to 6 or the nucleic acid molecule of claim 7.

9. The vaccine composition of claim 8, comprising the peptide of any one of claim 1 to 6 and an adjuvant.

10. The vaccine composition of claim 9, wherein the adjuvant is melanin.

11. The peptide of any one of claim 1 to 6, the nucleic acid of claim 7 or the vaccine composition claim 9 or 10 for use as a medicament or as a vaccine.

12. The peptide of any one of claim 1 to 6, the nucleic acid of claim 7 or the vaccine composition claim 9 or 10 for use for the treatment of cancer.

13. The peptide of any one of claim 1 to 6, the nucleic acid of claim 7 or the vaccine composition claim 9 or 10 for use according to claim 12, wherein the cancer is selected from the group consisting of glioblastomas, gliomas, melanomas, lung head-and-neck, cervical and testis cancers.

14. The peptide of any one of claim 1 to 6, the nucleic acid of claim 7 or the vaccine composition claim 9 or 10 for use according to claim 12, wherein the cancer is a low or high grade glial tumor.

15. An *in vitro* method for detecting, priming or stimulating CD8 lymphocytes directed against PTPRZ1, comprising contacting a peptide according to any one of claims 1 to 6, a nucleic acid molecule according to claim 7, optionally with an adjuvant, with antigen presenting cells and T-lymphocytes.
